# EUROPEAN PATENT APPLICATION

(11) **EP 3 916 078 A1**
(43) Date of publication of application: **01.12.2021**
(21) Application number: 21173872.9
(22) Date of filing: 14.05.2021
(51) Int. Cl.: C12M 1/00

(54) **AUTONOMOUS PHOTOBIOREACTOR**

(30) Priority: 26.05.2020 IT 202000012385
(71) Applicant: Tem Electtromeccanica S.r.l., 24050 Grassobbio (BG) (IT)
(72) Inventor: BURGASSI, Alessio, 24050 TEM ELETTROMECCANICA S.r.l., Grassobbio (BG) (IT); CRIPPA, Marco Maria, 24050 TEM ELETTROMECCANICA S.r.l., Grassobbio (BG) (IT)
(74) Representative: Capasso, Olga

(57) **Abstract**

An autonomous apparatus (1) for growing photosynthetic microorganisms, comprising a culture tank (10) configured to contain photosynthetic microorganisms immersed in an aquatic environment, said culture tank (10) consisting of a hollow main body (12) extending along a longitudinal axis (13) and a base (14) attachable to a first end (121) of the main body (12), wherein the base (14) comprises at least one drainage opening (141) for the release of the content of the culture tank (10), a lid (20) attachable to a second end (122) of the main body (12) provided with at least one inlet opening (22) for introducing water, a compression unit (30) coupled to the base (14) of the culture tank (10) for introducing at least one pressurised gas flow into the culture tank (10), and a control unit (40) for managing the operation of the apparatus (1) according to a set programming, wherein the apparatus also comprises first lighting means (50), insertable inside the culture tank (10), and second lighting means (60), positioned outside the culture tank (10) along the main body (12), to homogeneously light the microorganisms during the growth phase.

## Description

### TECHNICAL FIELD

This invention relates to an autonomous apparatus for growing microorganisms, preferably photosynthetic microorganisms, such as microalgae, cyanobacteria, and photosynthetic bacteria. In particular, it relates to a photobioreactor. In addition, this invention relates to a plant comprising one or more of said autonomous apparatuses. In addition, this invention relates to the use of said apparatus for cultivating photosynthetic microorganisms.

### PRIOR ART

A photobioreactor (FBR) is an apparatus comprising a hydroponic culture tank that is usually positioned vertically. Apparatuses of this kind are used for growing photosynthetic microorganisms such as microalgae, cyanobacteria, and photosynthetic bacteria. They are mainly housed inside covered structures that may or may not be exposed to sunlight. In this case, plexiglass or glass greenhouses or industrial or agricultural salvage sheds can be used.

The function of an FBR is to create a controlled environment that is as isolated as possible from the surrounding environment in order to ensure a controlled process in which it is possible to grow the biomass being cultivated.

FBRs are distinguished from tank cultivation; they differ in the absence of contact (in whole or in part) with the external environment.

Known photobioreactors represent simple passive elements of fixed plants and depend on each other and on various elements that comprise the plant. Therefore, in a modular design of a plant comprising several photobioreactors, the increase in the number of FBRs involves not only the downsizing of the fixed plant (piping and electrical cables), but also the implementation of all those systems necessary for the operation of the FBRs themselves, such as air blowing and temperature control.

In addition, the photobioreactors are usually elements of a complex plant that need continuous adjustments. Currently, these take place via FBR blocks, thus making the specific calibrations that are needed to optimise the cultures present in each reactor difficult.

An essential element for growing and developing the culture is light. For this reason, all photobioreactors are equipped with an autonomous lighting system that is generally positioned inside the culture tanks, or outside them. However, systems positioned exclusively inside or exclusively outside the tank do not always ensure homogeneous and even lighting of the culture.

Another essential element is undoubtedly the movement of water. For example, for a good culture of photosynthetic microorganisms such as microalgae, the natural habitat must not be stagnant. Typically, the water supply systems of photobioreactors are supported by air injectors (air blowers) that blow vertically from the bottom upwardly. This results in an even linear thrust of the air, when it comes into contact with the water, and does not ensure optimal mixing of the biomasses inside the tank. Said remixing is, instead, necessary to ensure uniformity and homogeneity of the culture's exposure to light.

One purpose of this invention is to overcome some or all of the above-mentioned drawbacks of known systems and to provide apparatuses for growing photosynthetic microorganisms that are compact, effective, and easy to maintain.

### DESCRIPTION OF THE INVENTION

In a first aspect of the invention, an autonomous apparatus for growing photosynthetic microorganisms is presented. The apparatus may, in detail, be represented by a photobioreactor.

The apparatus comprises a culture tank configured to contain photosynthetic microorganisms immersed in an aquatic environment. The culture tank consists of a hollow main body extending along a longitudinal axis and a base attachable to a first end of the main body, wherein the base comprises at least one drainage opening for releasing the contents of the culture tank. For example, the main body may have a cylindrical shape while the base may have a hemispheric shape attached to one end of the cylindrical structure of the main body. In detail, the diameter of the base coincides with the diameter of the cylindrical structure. The main body may be a modular structure that can vary in height from a minimum value, for example 2 m, to a maximum value, for example 4 m. The main body may consist of a plurality of hollow elements, for example cylinders, which are attached to each other by appropriate fastening means. The hollow elements may be attached to each other by means of screw connections and a washer placed between these to ensure the culture tank has a watertight seal. All the elements that make up the main body can be made of transparent thermoplastic material that is highly compatible with food. The material used may have a transparency similar to that of glass in order to allow clear inspection of the state of the culture contained within the tank and to allow light to pass through it.

The apparatus also comprises a lid attachable to a second end of the main body, provided with at least one inlet opening for introducing water. The lid represents the closure of the culture tank. In the case of a main body with a cylindrical cross-section, the lid will have a disc shape, so that the diameter of the cylindrical cross-section corresponds to the diameter of the disc. The lid can be made of Polymethyl Methacrylate (PMMA) and all the motors required for the movement of the various parts of the apparatus can be housed on it. The lid may comprise a plurality of vents, for example four, dedicated to collecting both unused ("consumed") carbon dioxide from the microorganisms inside the tank and condensation generated inside the culture tank.

As will be seen below, these vents can also be arranged to allow external elements (washing units) to be inserted inside the culture tank.

In addition, the apparatus comprises a compression unit coupled to the base of the culture tank to introduce at least one pressurised gas flow into the culture tank and a control unit to manage the operation of the apparatus according to a set programme. The compression unit (or compressor) is a piece of equipment for generating and introducing a flow of gas, e.g., air and/or carbon dioxide, into the tank at a given pressure. The compression unit is equipped with controlled resistors for thermoregulating the gas mixture introduced. The pressure of the gas mixture released from the compression unit can vary between 0.2 bar and 2 bar depending on the requirements of the culture. The adjustment can be done either by means of computerized digital controls or manual ones. The control unit can be represented by a system comprising hardware and software able to autonomously manage the apparatus. The control unit may comprise a processor for receiving via a network a program with which it is possible to adjust the growth cycle of each cultivated culture, autonomously setting the water temperature, the air and/or carbon dioxide flow, and the lighting for each day of the growth period of the culture. Optionally, the control unit comprises a control panel positioned outside the tank to allow a user to enter data and observe the pressure or temperature values inside the tank via a display.

In particular, the apparatus comprises first lighting means, or internal lighting means, which can be inserted (insertable) inside the culture tank and second lighting means, or external lighting means, positioned outside the culture tank along the main body to homogeneously light the microorganisms during the growth phase from both inside and outside the culture tank. The first (internal) lighting means have the task of providing lighting from inside the tank towards the outside while the second (external) lighting means have the task of providing lighting from outside the tank towards the inside. This is, of course, possible due to the transparent material of which the main body of the culture tank is made. The use of both internal and external lighting significantly increases the performance of the apparatus compared to known systems. In fact, the optical distance that is generated when the culture develops declines until it reaches 100 mm. Therefore, a single lighting source (whether internal or external) will never ensure homogeneous illumination within the culture. If one were to compensate for the reduction in optical distance due to the use of a single lighting source (internal or external) by increasing the power of the light sources, one would run the risk of irreparably damaging, or "burning", the culture. It should also be noted that the use of a reflective structure in place of one of the light sources (e.g., reflective panels placed outside the tank and facing the centre of the central body to reflect light from a light source placed in the centre of the tank) could never ensure a homogeneity of lighting inside the tank that would be comparable to the use of two separate light sources, one internal and one external.

In one example, the second (external) lighting means comprise a plurality of second light sources extending along the main body of the culture tank and rotatable about an axis parallel to the longitudinal axis of said main body so as to vary the lighting orientation and angle of the second light sources towards the culture tank. For example, the second light sources may be LED lamps positioned outside the culture tank. To vary the intensity and nature of the lighting, the second light sources may be coated, or not, with opalised lenses. In particular, there may be two or more second light sources positioned at an equal distance from each other around the outer perimeter of the main body of the culture tank. Each second light source may be configured to rotate by a rotation angle, for example 180°, on its axis so as to irradiate the inside of the culture tank with direct light or with reflected light. The rotation axis is essentially parallel to the longitudinal axis of the main body of the tank. In other words, in the case of a vertically arranged culture tank, the second light sources will also extend vertically. For example, the apparatus may comprise four lamps positioned opposite each other in pairs on the vertical and horizontal axes of the tank. They can be rotated either manually or via the control of motors controlled by a processor placed in the control unit.

The light intensity and spectrum of the second light sources can be adjusted by means of motorised movement from the control unit or manually. Motorised control can take place automatically using a processor independently of the internal lighting means.

The total power of the entire lighting system (internal and external lighting means) can vary between a minimum of 400 Watts and a maximum of 800 Watts.

According to one example, every second light source of the second (external) lighting means are attachable between a first rotatable support insertable in the lid and a second rotatable support insertable in the base of the culture tank. In this way, the second light sources (lamps) can be easily replaced without affecting the rotating operation thereof. The first and second supports can be advantageously attached by means of appropriate seats in the lid and base of the tank respectively.

In one example, the second lighting means can advantageously comprise eight second light sources (lamps) positioned equidistant from each other and extending along the main body of the culture tank. For example, the lamps can be LED bars each of which is divided into two independent circuits so as to allow proper illumination regardless of the type of algae being cultivated.

In one example, the first lighting means may comprise a plurality of first light sources (lamps) that are attachable to a longitudinal structure insertable inside the culture tank. For example, the structure can be composed of a single triangular bar on whose sides LED lamps are installed, which also have two independent channels (like the external ones). Each LED lamp can have an illumination angle of 120° so that with a trigone you can cover 360° of a complete circumference.

According to one example that can be combined with the preceding examples, the main body can have a cylindrical shape with a diameter that ranges between 550 mm and 450 mm, preferably of 500 mm, and the first lighting means may be inserted in a container extending along the longitudinal axis of the main body, which also has a cylindrical shape with a diameter that ranges between 250 mm and 150 mm, preferably of 100 mm. It should be noted that the apparatus has been designed to eliminate the shadowy areas that are created inside the culture tank when algae growth reaches the "productive" level. Following various studies, it was found that the optical distance that a light source can travel in this cultivation situation does not exceed 100 mm. Therefore, by using an external cylinder (culture tank) with a diameter of 500 mm and an internal one (internal lamp holding cylinder) with a diameter of 200 mm, an optical distance of only 75 mm is obtained, ensuring that no unlit areas inside the culture tank are ever created. In fact, the distance between the walls of the two cylinders is defined by 500 mm - 200 mm / 2 = 150 mm, while the distance travelled by each light source is defined by 150 mm / 2 = 75 mm.

In other words, the first lighting means are arranged parallel to the second lighting means, in which the distance between the first lighting means and the second lighting means ranges between 200 mm and 100 mm, and is preferably 150 mm.

In one example, the container inside of which the first lighting means are inserted may comprise an upper anchorage point at the second end of the main body and a lower anchorage point at the drainage opening. For example, by means of a point-shaped connection pivotally joined directly inside the draining duct.

According to one example that can be combined with the preceding examples, the apparatus may also comprise a plurality of reflective panels connected to the second (external) lighting means extending along the main body of the culture tank and attached between a seat positioned in the lid and a seat positioned in the base of the culture tank. For example, each reflective panel may be curved and positioned at each individual second light source of the external lighting means. Each panel can be configured in such a way as to enable homogeneous diffusion of the light that is projected against it by the second light sources when these are turned towards the culture tank. The reflective panels of the second (external) lighting means improve the operation of the apparatus. These, in fact, are integrally attached between the base of the culture tank and the lid and have a twofold function: to make the apparatus structurally solid and to reflect (the face turned towards the culture tank is mirrored) the light emitted by the second light sources so that they always generate a homogeneous luminous flux towards the culture tank and nothing is dispersed in the environment creating light pollution.

According to one example that can be combined with the preceding examples, the apparatus also comprises a plurality of sliding reflective panels extending along the main body of the culture tank and sliding along the outer perimeter of said main body and wherein the external lighting means are positioned in a space between said sliding reflective panels (i.e. between said fixed reflective panels) and said outer perimeter of the main body. In particular, the sliding reflective panels define a space with the main body of the culture tank inside of which the second light sources of the second (external) lighting means are positioned. Reflective panels of this type are used to create an area of maximum reflection since the areas not covered or only slightly covered by fixed reflective panels can be closed off by sliding panels. For example, the sliding reflective panels can be positioned on trolleys that allow them to move and therefore to open and close according to the needs that arise (for example, to dissipate excessive heat generated by the external light sources or to create an environment with maximum reflection). The sliding reflective panels can be moved manually or in a motorized and automatic manner via the control unit. The movement of the sliding reflective panels can be used both to open (clear) the area in front of the culture tank in order to dissipate any excess temperature generated by the second (external) lighting means and to be able to inspect the culture inside the tank.

According to one example that can be combined with the preceding examples, the base of the culture tank has a hemispheric shape with the drainage opening centrally positioned on the apex of the hemisphere and comprises a plurality of nozzles for coupling with the compression unit equipped with an autonomous resistor. The nozzles are arranged radially with respect to the drainage opening and inclined by an angle α in relation to the longitudinal axis of the main body of the culture tank. For example, the angle α may vary between 15 and 30 degrees in relation to the longitudinal axis. The nozzles represent the so-called air blowers and are used to introduce air or carbon dioxide from the compression unit inside the culture tank. Advantageously, the nozzles are radially arranged on various levels of circumference, for example three. A check valve may be placed between each nozzle and the connection to the compression unit to prevent culture water from flowing towards the air-blowing system or the compression unit. At the base's lower vertex, i.e. at the drainage opening, there is a threaded connection that connects to the drain of the cultivated culture or biomass. The pulsating system for introducing air inside the culture tank (air blowing) produced with nozzles that are tilted both radially and axially, positioned inside a tank having the shape of a hemisphere, makes it possible to give the water (and, consequently, the algae contained in it) a continuous rotary movement. In this way, a continuous "remixing" of the algae is generated so that their irradiation varies continuously, ensuring that they alternate between situations of maximum brightness (when close to the surface of the cylinders) and situations of reduced brightness (when close to the central point of the optical distance between the two luminous points).

According to one example, the apparatus also comprises an air separator positioned between the compression unit and the base of the culture tank for dividing the introduction of air inside the tank. In particular, the air separator is connected to the control unit to allow sequential introduction of gas into the culture tank through a selected plurality of nozzles. This significantly improves the efficiency of the apparatus. Usually, the air flows into the culture tank simultaneously from all the nozzles, resulting in a constant upward thrust from the bottom (vertically or tilted, depending on the position of the nozzles). By using the air separator instead, it is possible to create a sort of "wave motion" as only one row (or several opposite rows) of the nozzles introduce air in sequence inside the tank. For example, a sequence of air introduction and air introduction interruption may be determined so as to generate pulsating motion that, in turn, will impart a rotational motion to the content of the culture tank. The presence of the air separator therefore increases the rotational motion inside the tank. The air separator can be controlled by a processor via the control unit to manage the sequence in which the nozzles are opened or closed.

According to one example that can be combined with the preceding examples, the compression unit comprises a first inlet for introducing atmospheric air, a second inlet for introducing carbon dioxide, and at least one valve with electronic control connected to the control unit for adjusting the amount of atmospheric air and/or carbon dioxide introduced into the culture tank.

In a second aspect of the invention, a modular plant is presented. The modular plant for growing photosynthetic microorganisms comprises two or more autonomous apparatuses according to the first aspect of the invention. Each apparatus is provided with an inlet water connection for introducing water into the culture tank directly through the inlet opening of the lid, an outlet water connection for draining the culture tank through the drainage opening of the base, and an electrical connection for powering the control unit and all other electrical components of the apparatus.

In this way, the apparatus for growing photosynthetic microorganisms, i.e. the photobioreactor, is completely autonomous and represents a stand-alone modular element able to operate with a simple connection to the water mains (for the input of water and the draining of the culture) and to the electricity mains (for supplying power to all electronic devices, in particular the control unit). In this way, the size of the installed plant is variable and is not predefined in the initial design phase as it is always possible to enlarge the original plant (useable surfaces permitting) simply by connecting one or more additional apparatuses (photobioreactors). This is possible because each photobioreactor is equipped with all the necessary components for its autonomous operation and is connected to the plant to which it belongs only for introducing water (in this case, any rubber tube could be suitable for the purpose), draining the tank (in this case too, any rubber tube could be suitable for the purpose), and the electrical connection.

The maintenance system, according to one example, comprises a washing unit that can be coupled to an apparatus according to one of the preceding embodiments. In particular, the washing unit comprises a plurality of telescopic arms that can be inserted inside the culture tank through a plurality of openings positioned on the lid of the apparatus. Each arm is telescopic and consists of a set of elements that extend lengthwise. At one end, each arm is equipped with a rotating cleaning unit that can be inserted into the culture tank. At a second end, each arm is provided with a connector for introducing a pressurised cleaning fluid.

In a third aspect of the invention, the use of one or more autonomous apparatuses, according to one of the preceding claims, for cultivating photosynthetic microorganisms, is presented. For example, photosynthetic microorganisms may comprise spirulina, chlorella, haematococcus pluvialis, or similar microorganisms.

These and other aspects of this invention will become clearer in the light of the following description of some preferred embodiments described below.
Fig. 1 shows a schematic representation of the apparatus according to one example;
Fig. 2a-c show a schematic perspective representation of the apparatus according to one example;
Fig. 3 shows a schematic representation of the apparatus support element according to one example;
Fig. 4a-c show a schematic representation of the apparatus compression unit according to one example;
Fig. 5a-b show a schematic representation of the apparatus culture tank according to one example;
Fig. 6a-c show a schematic representation of the second or external lighting means (5a) and first or internal lighting means (5b-5c);
Fig. 7a-b show a schematic representation of the washing unit according to one example;
Fig. 8a-b show two instances of the washing unit's movement; and
Fig. 9 shows a plant for growing photosynthetic microorganisms.

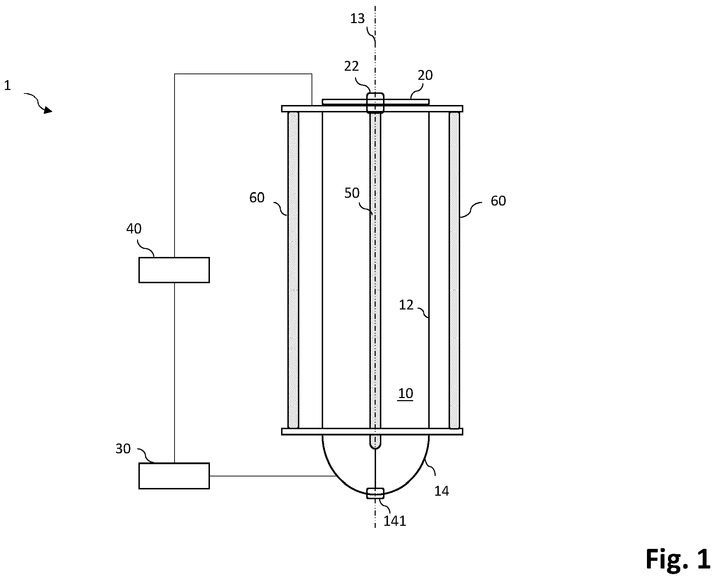

Figure 1 shows, in a completely schematic manner, the apparatus 1 according to one example. The apparatus 1 is suitable for growing photosynthetic microorganisms and comprises a culture tank 10 consisting of a hollow main body 12 extending along a longitudinal axis 13 and a base 14 that can be attached to one end of the main body 12. The base 14 comprises a drainage opening 141 for releasing the content of the culture tank 10. The apparatus 1 also comprises a lid 20 that can be attached to another end of the main body 12 provided with an inlet opening 22 for introducing water. In addition, the apparatus comprises a compression unit 30 coupled to the base 14 of the culture tank 10 to introduce at least one pressurised gas flow into the culture tank 10 and a control unit 40 to manage the operation of the apparatus 1 according to a set programme.

The apparatus 1 additionally comprises two different and separate lighting means. In particular, the apparatus 1 comprises first lighting means 50 that are inserted inside the culture tank 10 and second lighting means 60 positioned outside the culture tank 10 along the main body 12 to homogeneously light the microorganisms during the growth phase. It should be noted that there may be more than one second lighting means 60. For example, Figure 1 depicts at least two second lighting means 60 positioned equidistant outside the culture tank 10. Both the first lighting means 50 and the second lighting means 60 extend longitudinally along or parallel to the longitudinal axis 13.

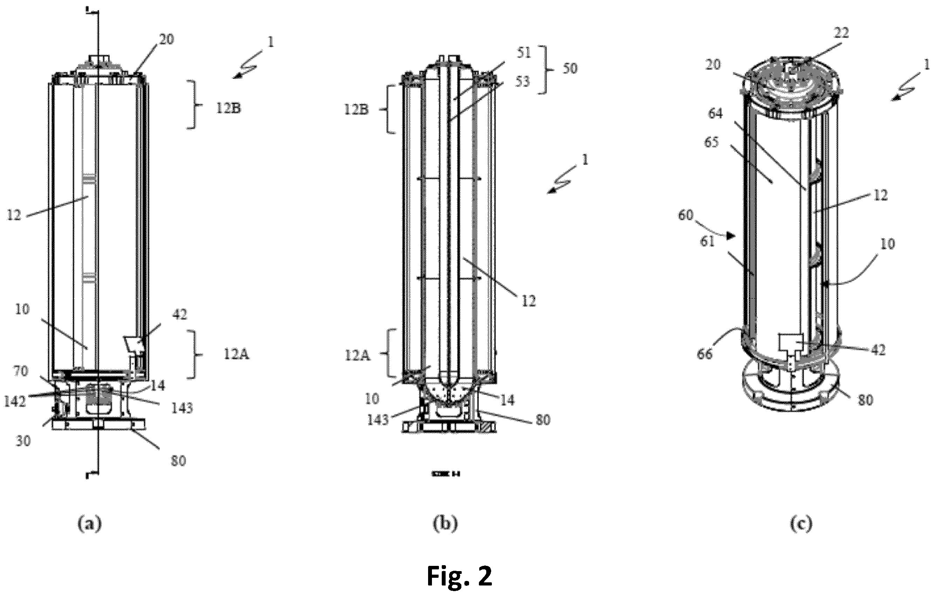

Figure 2a represents a more detailed schematic representation of the apparatus 1 according to a side view, and Figure 2b represents the vertical cross-section of the apparatus 1 shown in Figure 2a along the D-D line.

As illustrated in Figure 2a, the apparatus 1 comprises a vertically positioned culture tank 10 that consists of a main body 12 with a cylindrical shape and a base 14 with a hemispheric shape 143 attached to the main body 12 in its lower portion 12A. The base 14 comprises a plurality of nozzles 142 arranged radially thereon. In contrast, a lid 20 is positioned at the upper portion 12B of the main body 12 to close the culture tank 10. The apparatus 1 rests on a support element 80 to stabilise its vertical position. A compression unit 30 is attached to the lower part of the apparatus 1 and coupled to the base 14 of the culture tank 10 via nozzles 142 for introducing pressurized air into the tank 10. In addition, there is an air separator 70 positioned downstream of the compression unit 30 and upstream of the base 14 of the tank 10 for distributing the air introduced inside the tank 10. At the lower portion 12A of the main body 12 of the tank 10, the apparatus 1 comprises an electrical panel 42 forming part of a control unit 40 so that a user can input control data of the apparatus 1. Figure 2b more clearly illustrates the inner region of the apparatus. In particular, this figure clearly shows that the tank 10 consists of a hollow main body 12 extending along a longitudinal axis 13 and a base 14 at the lower portion 12A of the cylindrical main body 12. In addition, Figure 2b shows the presence of first lighting means or internal lighting means 50 positioned in the centre of the main body 12.

Figure 2c shows a perspective representation of the apparatus 1. In detail, the figure shows that the tank 10 consists of a main body 12 with a cylindrical shape extending longitudinally and representing the "core" of the apparatus 1. In the upper part, there is a lid 20 that serves to close the tank 10 and that therefore represents the apex of the apparatus 1. The lid 20 comprises a plurality of seats or openings for all possible automated movements and for attaching any ducts to collect both air condensation and carbon dioxide escaping from the culture tank 10. Using the latter openings, it is possible to access the inside of the culture tank 10 with a special washing unit 90 described below. In particular, the lid comprises at least one opening 22 for introducing clean water inside the culture tank 10.

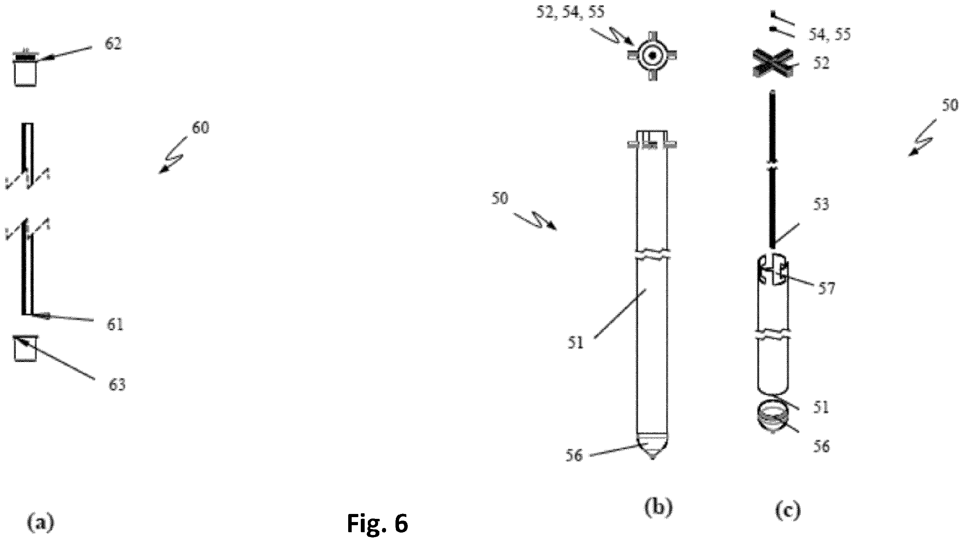

Figure 2c also shows the arrangement of second lighting means or external lighting means 60 and of reflective panels 64, 65. In detail, said external lighting means 60 comprise four LED lamps 61 arranged at 90 degrees to each other (the figure basically only shows one of them), attached within special supports 62, 63 (shown in Figure 6a) that have their rotation fulcrum in the base 14 of the culture tank 10 and in the lid 20. The apparatus 1 also comprises fixed reflective panels 64 and sliding reflective panels 65. From the figure, it should be noted that the sliding reflective panels 65 have a face turned towards the culture tank 10 that can be mirrored. Like the fixed reflective panels 64, they perform the function of reflecting light back towards the culture tank 10. Unlike the fixed panels 64, the sliding panels 65 are not integrally attached to the structure of the apparatus 1 but can slide on trolleys 66 that enable them to be opened and closed along the external perimeter of the apparatus 1, i.e. along the external perimeter of the main body 12 of the tank 10. Figure 2c shows that the lamps 61 are located in a space between the main body 12 of the tank 10 and the fixed reflective panels 64 and the sliding ones 65.

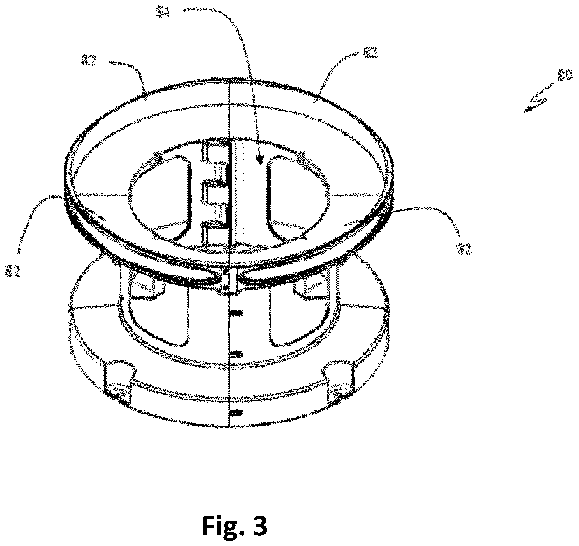

Figure 3 shows the support element 80 on which the culture tank 10 rests. The support element 80 is composed of four circular-portion elements 82 that are joined together by means of joints and screw connections. Each element 82 is made so that it provides an interlocking coupling with the above components that are attached using screw connections. The support element 80 comprises an upper circular opening 84 that serves to receive the base 14 of the culture tank 10, as noted in Figure 2a. In addition, the support element 80 may comprise additional openings and special seats for the passage and placement of cables and piping.

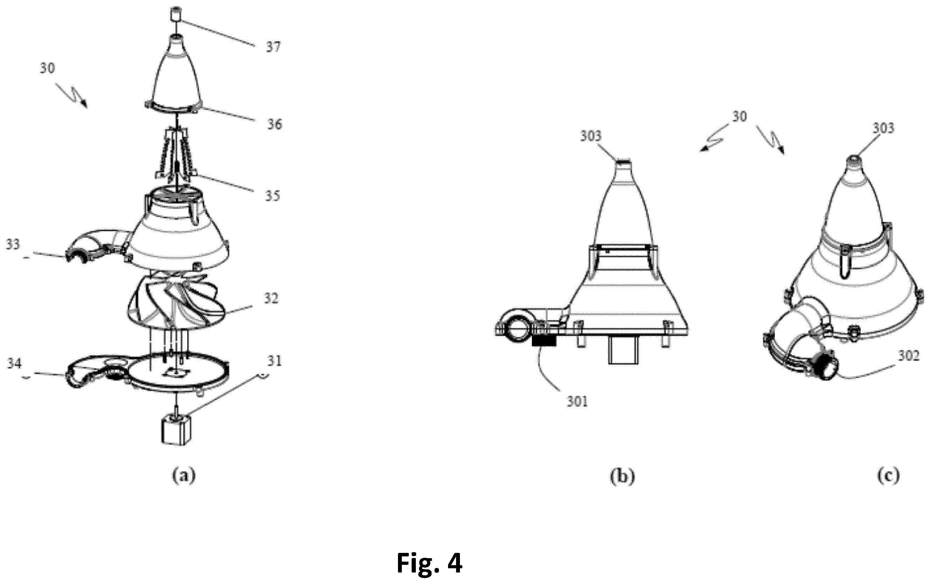

Figure 4a shows the compression unit 30 in an exploded view. In particular, the compression unit 30 consists of a motor 31, a rotor 32, an upper cover 33 of the rotor 32, a base cover 34 of the rotor 32, an electrical resistor 35 with a corresponding temperature sensing probe, an upper closure element 36, and a connection 37 for inserting an air tube. All the components of the compression unit can be attached to each other by means of connections such as screws, glues, welding, etc. Once joined together, they create a structure like the one shown in Figures 4a and 4b. The compression unit 30 has two air inlets: a first inlet 301 for atmospheric air and a second inlet 302 for carbon dioxide. Ball valves (not shown in the figure), with electronic control, can be attached to each of these inlets to adjust the amount of air and/or CO₂ that is introduced into the culture tank 10.

During its flow, the air passes through the rotor 32 that, due to its rolling shape and rotary motion, imparts energy to the air mixture. The air mixture accelerated by the rotor 32 passes through the resistors 35 heating up. The compression unit 30 may be fully controlled by a processor in the control unit 40 (with respect to both the speed of the rotor 32 and the temperature of the resistors 35). In the absence of resistors 35, the speed of the rotor 32 is fixed (2 preset speeds).

A tube may be inserted into the connection 37, terminating, for example, in the inlet air connector of the air separator 70, when present.

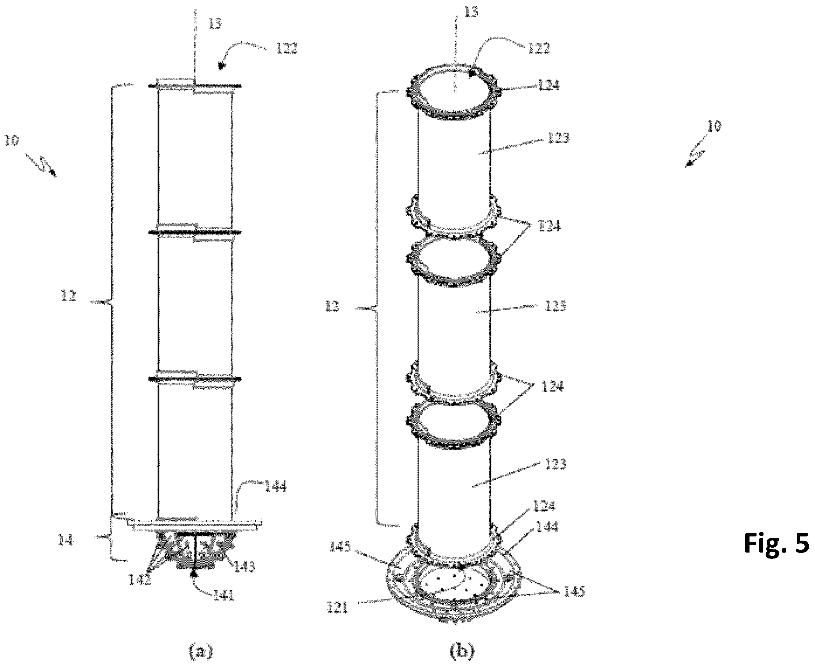

Figures 5a and 5b show the culture tank 10 viewed from the side (Fig. 5a) and in perspective in an exploded view (Fig. 5b). The culture tank is configured to contain the cultivated biomass and consists of a hemispheric base 143 and a cylinder 12 (or main body) composed of a plurality of elements 123 (in this case 3 modules with a nominal outer diameter of about 500 mm and an approximate height of about 3 m). The elements 123 are attached to each other by means of connecting means, for example screws, at the joint collars 124 at the two ends of the individual elements 123. The cylinder 12 comprises a first end 121 at which the base 14 of the tank 10 is attached and a second end 122 at which the lid 20 is positioned. The hemispheric base 14 comprises a plurality of nozzles or air blowers 142 arranged radially on the surface of the hemisphere 143. Finally, at the apex of the hemisphere 143 there is the drainage opening 141 of the tank 10. The base 14 of the tank 10 comprises a collar 144 connecting with the main body 12 and, in particular, with the joint 124 of its lower element 123. The collar 144 comprises a plurality of seats 145 into which one end of the second light sources 61 of the external lighting means 60 is inserted (the other end of the second light sources 61 can, instead, be inserted inside a special seat present in the lid 20, which is not shown in the figure). It should be noted that the ends of the second light sources 61 are inserted by means of special supports as described below. The collar 144 may also comprise additional seats for inserting, fixing, or sliding the reflective panels 64 and 65.

Figure 6a shows a detail of the external lighting means 60. In particular, the second light source 61 is represented by an LED lamp extending longitudinally between a first rotating support 62 and a second rotating support 63. The first support 62 has the rotation fulcrum in the lid 20 while the second support 63 has its rotation fulcrum in the base 14 of the tank 10, i.e. in the collar 144. Each of the lamps 61 can be rotated so as to best orient the luminous flux they emit in order to optimise the lighting of the culture. The rotational movement may be controlled manually or automatically using motors controlled by the control unit 40.

Figures 6b and 6c show the structure of the internal lighting means 50. In particular, the internal lighting means 50 comprise a containment cylinder 51 and a lamp 53 inserted inside said cylinder 51. The cylinder 51 is made of transparent (or opalised) thermoplastic material (PMMA) that is highly compatible with food. The cylinder 51 is configured to contain the internal lamp 53, isolate it from the water, and create a space between the biomass and the irradiation point in order to prevent the lighting from burning the cultivated content. The connection between the tank 10 and the cylinder 51 is watertight so as to prevent water vapour's passing from the tank 10 to the inside of the containment cylinder 51 and thus prevent possible malfunctions or breakages of the lamp 53 contained therein. The base of the cylinder 51 is fixed by a closure 56 made of the same material as the cylinder 51. This closure 56 serves to seal the cylinder 51 from the water contained in the culture tank 10 and, at the same time, acts as a support base for the internal lamp 53. The internal lamp 53 is fixed by a screw connection 54, 55 to a support beam 52 and, by means thereof, is fitted inside the cylinder 51 so as to create a structure integral therewith. For this purpose, the upper part of the cylinder 51 is provided with special slots 57 for interlocking. The lamp 53 is fixed by means of a quick coupling with the lid 20 to essentially prevent the lamp 53 from being lifted by the thrust generated by the water inside the tank 10. To further reduce the upward thrust of the cylinder 51 by the water, the closure 56 has an ogival shape. The lamp 53 positioned inside the cylinder 51 comprises three faces arranged at 120° from each other in order to ensure homogeneous lighting inside the culture tank 10.

The intensity and light spectrum of the lamp 53 may be manually adjusted or automated using motors controlled by the control unit 40. This can be done independently of the adjustment of the intensity and light spectrum of the external lighting means 60.

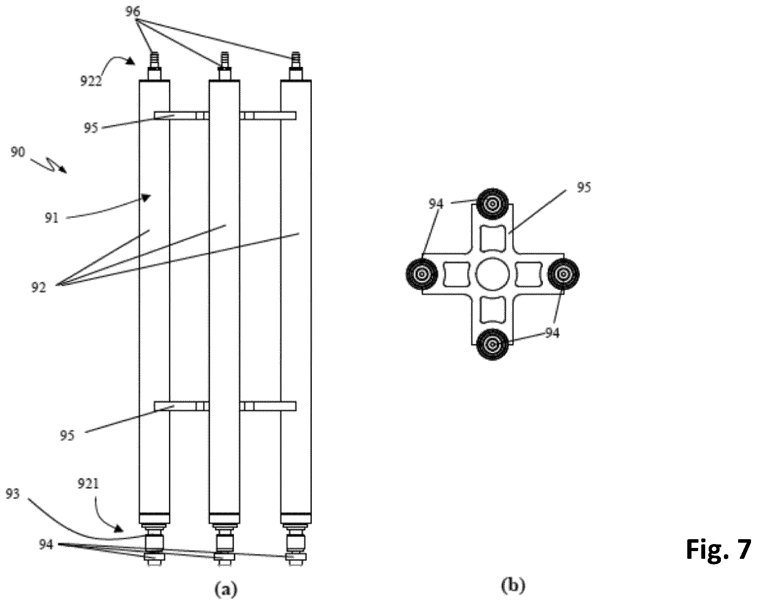

Figures 7a and 7b show the washing unit 90 - viewed from the side and from below - for an apparatus 1 maintenance system. In order to ensure that the cultivated biomass is of a high quality, the apparatus 1 must be cleaned periodically. Since the culture tank 10 is practically sealed, the washing unit 90 is provided with four telescopic arms 93 that are inserted inside the culture tank 10 by means of the special openings located on the lid 20. In particular, Figure 7a illustrates the external structure 91 of the washing unit that consists of four rigid cylinders 92 connected to each other by connecting means 95. In operation, the external structure 91 is positioned on the lid 20 of an apparatus 1 at special openings.

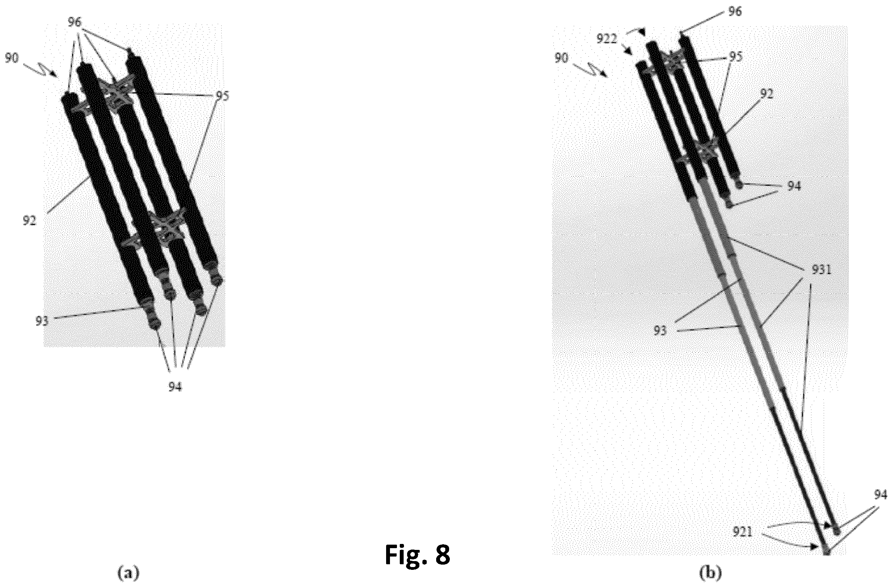

Each arm 93 comprises a set of telescopic elements 931 that extend longitudinally. At one end 922, each arm 93 is connected to a tube from which the pressurised cleaning fluid comes and, at the other end 921, to a special rotating cleaning head 94 designed for cleaning the tank 10. The pressure of the cleaning fluid activates the rotating heads 94 (one for each arm 92) that, thanks to their movement, clean the inside of the culture tank 10. The arms 93 and corresponding extensions 931 lower, by gravity, and are retracted by rewinding the irrigation tube. Specifically, Figures 8a and 8b show two instances of applying the washing unit 90 in which only two of the arms 93 are used for washing the tank 10. In Figure 8a, the arms 93 begin to extend while in Figure 8b, the arms 93 are at their maximum extension. Thanks to this washing unit 90, it is possible to reach every internal surface of the tank 10 without needing to remove the tank 10 from the apparatus 1.

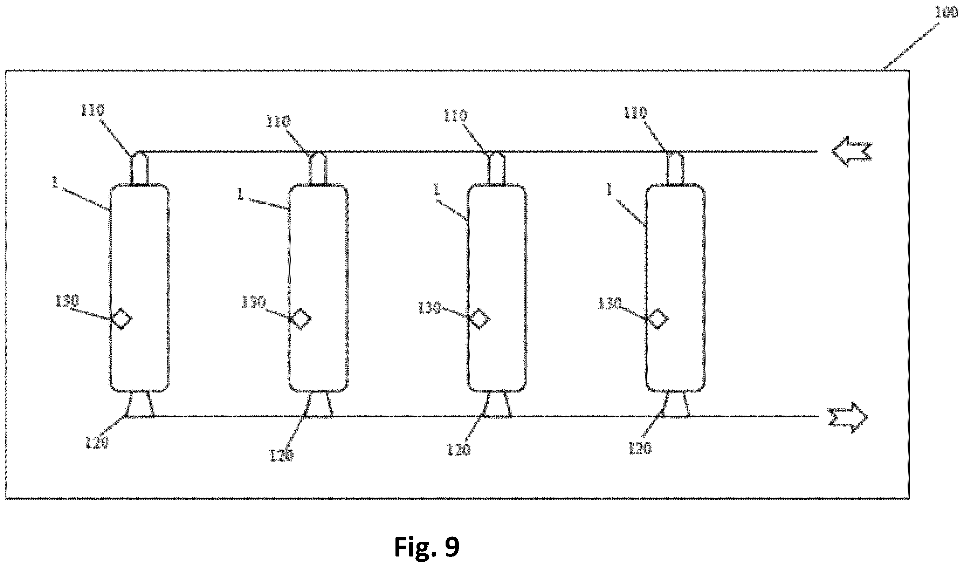

Finally, Figure 9 shows a schematic block representation of the modular plant 100 for growing photosynthetic microorganisms. Specifically, the plant comprises four apparatuses 1 arranged in series. It should be noted that each apparatus 1 is a stand-alone element capable of operating independently of each other. In particular, each apparatus 1 is only connected to the plant 100 for introducing water 110, for draining the tank 120, and for the electrical connection 130.

The following are non-limiting examples of cultivation of various types of photosynthetic microorganisms applying the apparatus according to this invention. In particular, the cultivation of spirulina, chlorella, and *haematococcus pluvialis* are considered.

### Spirulina

Spirulina is the generic name for a dried biomass that is obtained from collecting the so-called spirulina algae *(Arthrospira platensis).* This organism is incorrectly referred to as algae (since it does not have a leaf structure and is a prokaryote), when in fact it is a cyanobacterium. Spirulina is considered to be a food ingredient by the European Food Safety Authority (EFSA) and can be included in dietary supplements, food products, or used purely in cooking or cosmetics. The dietary supplements produced have the following indications:
- improved mental performance and concentration;
- help in sports activities and recovery from psycho-physical stress;
- promotes the natural organic defences; in this regard, it should be pointed out that two researchers have recently filed a recipe to increase the defences against viruses that attack RNA where spirulina is the first ingredient.

Dry spirulina contains approximately 60% (51-71%) protein. It has a complete protein content, i.e. comprising all the essential amino acids even if the content in methionine, cysteine, and lysine is substantially lower than in meat, egg, and milk proteins. The fat content of spirulina is about 7% by weight. Spirulina is rich in gamma-linolenic acid (GLA) and also provides alpha-linolenic acid (ALA), linoleic acid (LA), stearidonic acid (SDA), eicosapentaenoic acid (EPA), docosahexaenoic acid (DHA), and arachidonic acid (AA). Spirulina also contains vitamins B1 (thiamine), B2 (riboflavin), B3 (nicotinamide), B6 (pyridoxine), B9 (folic acid), vitamin C, vitamin D, vitamin A, and vitamin E. It is also a source of potassium, calcium, chromium, copper, iron, magnesium, manganese, phosphorus, selenium, sodium, and zinc. Finally, spirulina contains many pigments that can be beneficial, including beta-carotene, zeaxanthin, chlorophyll-a, xanthophyll, echinenone, myxoxanthophyll, canthaxanthin, diatoxanthin, 3'-hydroxyechinenone, beta-cryptoxanthin, and oscillaxanthin, as well as the phycobiliproteins c -phycocyanin and allophycocyanin.

### Chlorella

Chlorella is a microscopic freshwater green alga of the Chlorophyceaen class, believed to be one of the earliest forms of life on Earth. Its very high concentration of chlorophyll promotes antioxidant action. It is grown in ponds rich in mineral salts and is well known for its purifying and healing action due to its high level of nutrients and chlorophyll. In chlorella, it is possible to find an extremely high concentration of chlorophyll, from 10 to 100 times more than that which is generally found in green leafy vegetables. Chlorella is made up of 60% fully absorbable noble vegetable proteins and contains all the essential amino acids, the CGF (Growth Factor) that helps increase a sports person's muscle mass. It contains vitamins and numerous minerals such as iron, calcium, phosphorus, and micronutrients that have an important function in the general nutrition mechanism, promoting rapid energy recovery. Chlorella can also be used as a supplement for sports; it is able to increase the body's physical resistance and adaptability in environmental situations of overload and extraordinary stress, in order to improve overall efficiency. It is also particularly rich in zinc and other minerals, and the concentration of vitamin A (beta-carotene) is much higher than what can be found in green leafy vegetables: this is an extremely significant component in giving chlorella the power to strengthen the immune system, as well as being a valuable aid in detoxifying the blood and tissues of harmful chemicals. Chlorella has, therefore, been proven to be a therapeutic substance in the following cases:
- strengthening the immune system;
- helping to heal wounds of all kinds and ulcers;
- helping to protect against toxic pollution;
- normalising the digestive and intestinal function;
- stimulating the growth and repair of tissues (due to the high amount of nucleic acids)
- slowing down tissue aging.

It is well known that, once the tissues of our body have been purified and regenerated, the whole organism is able to repair and regenerate itself, preventing or minimising the duration of diseases, especially those of a more serious nature.

### Haematococcus pluvialis

The bright pink colour of some animals, such as salmon, lobsters, and flamingos is a consequence of their feeding habits. Haematococcus pluvialis, a microalgae component of zooplankton that produces the carotenoid astaxanthin, is at the base of it all. *Haematococcus pluvialis* is known to be the largest natural producer of this carotenoid, which gives the tissues of fish and crustaceans that feed on it (and flamingos, which, being at the top of the food chain, feed in turn on crustaceans) their bright pink colour. *Haematococcus pluvialis* is a unicellular green microalgae belonging to the Chlorophyceaen class. It commonly lives in the fresh water of lakes, rivers, and puddles and is characterised by a complex, but at the same time fascinating, life cycle. *Haematococcus pluvialis* can be found in four types of cell morphologies: macrozoid, microzoid, palmella, and hematocyst (or aplanospore). When growth conditions are favourable, *haematococcus pluvialis* is found mainly in the vegetative state in the form of macrozoids (spherical, green, biflagellate cells), which are characterised by high motility. In this state, macrozoids can divide by mitosis to generate up to 32 daughter cells (microzoids) within the cell wall of the mother cell. When, in contrast, stressful conditions occur, such as excessive light intensity, excessive salinity, nutrient deficiency, or anything else that can lead to oxidative stress, the macrozoids begin to lose their flagella, stop dividing, and undergo the so-called "encystment" process. Their cell wall begins to thicken (palmella stage), until it forms an ultra-resistant coating composed of a trilaminar sheath and a secondary wall resistant to acetolysis that allows them to survive extreme chemical and physical conditions. At this point, palmella enters the hematocyst (or aplanospore) stage and begins to synthesize and accumulate high amounts of carotenoids within lipid bodies in order to protect itself from irradiation damage and oxidative stress. Carotenoids are in fact able to neutralise free radicals and ROS, thanks to their molecular structure, which is characterised by long terpene chains with conjugated double bonds. Among all the carotenoids, astaxanthin is the one produced in the greatest quantity (up to 99% of total carotenoids under certain conditions) by *haematococcus pluvialis,* which is able to accumulate up to 6% of its dry weight in astaxanthin, acquiring a shimmering bright red colour. This ability makes *haematococcus pluvialis* unique and of enormous industrial interest in terms of the production of this molecule that has many applications in the food, nutraceutical, pharmaceutical, and cosmetic sectors. Astaxanthin is, in fact, a powerful antioxidant (its oxygen free radical scavenger capacity is 6000 times higher than vitamin C) and it has photo-protective and anti-inflammatory properties. It is able to prevent and limit lipid peroxidation by protecting cell membranes, while some studies attribute it with soothing properties for gastric ulcers and the power to protects against cardiovascular and neurodegenerative diseases. It is mainly used as a dietary supplement and in feed production in fish farms in order to achieve the desired red pigmentation of fish and shellfish. Numerous studies are also underway to develop astaxanthin-based drugs for the diseases listed above and to protect the skin from UV damage. The production of *haematococcus pluvialis* is entirely based on cultivating this valuable microalgae in photobioreactors.

Within them, cells in the vegetative state are initially cultivated under optimal conditions to promote their growth. When maximum cell density is reached, combined environmental stressors, such as nutrient deprivation and high light irradiation, are applied in order to induce astaxanthin accumulation, which maximum peaks within 3-5 days. At this point, the biomass is collected and processed in order to extract astaxanthin using organic solvents.

Based on this, in order to obtain astaxanthin it is necessary to generate a state of stress in haematococcus pluvialis: a state of stress that can be induced by a combination of external factors, such as nutritional factors and lighting of the external environment.

The apparatus 1 has been configured to best meet this need; thanks to all its automated systems, it can dramatically increase or decrease the amount of CO₂ introduced into the culture tank 10. Another feature of the apparatus 1 is the possibility of rotating the lamps of the external lighting system 60 so as to make them point directly towards the culture tank 10 increasing the intensity of the luminous flux turned towards the cultivated biomass.

Numerous additional modifications and variations can be made to the apparatus, plant, and use described above by a person skilled in the art with the aim of satisfying additional, contingent needs, all of which are comprised within the protective scope of this invention, as defined by the appended claims.

## Claims

1. An autonomous apparatus (1) for growing photosynthetic microorganisms, comprising:
a culture tank (10) configured to contain photosynthetic microorganisms immersed in an aquatic environment, said culture tank (10) consisting of a hollow main body (12) extending along a longitudinal axis (13) and a base (14) attachable to a first end (121) of the main body (12), wherein the base (14) comprises at least one drainage opening (141) for the release of the contents of the culture tank (10),
a lid (20) attachable to a second end (122) of the main body (12) provided with at least one inlet opening (22) for introducing water,
a compression unit (30) coupled to the base (14) of the culture tank (10) for introducing at least one pressurized gas flow into the culture tank (10), and
a control unit (40) for managing the operation of the apparatus (1) according to a set programming,
**characterised by**
first lighting means (50) insertable inside the culture tank (10) and second lighting means (60) positioned outside the culture tank (10) along the main body (12) to homogeneously light the microorganisms during the growth phase.

2. The apparatus (1) according to claim 1, wherein the second lighting means (60) comprise a plurality of second light sources (61) extending along the main body (12) of the culture tank (10) and rotatable about an axis parallel to the longitudinal axis (13) of said main body (12) so as to vary the orientation and angle of the lighting of the second light sources (61) towards the culture tank (10).

3. The apparatus (1) according to claim 2, wherein each second light source (61) of the second lighting means (60) are attachable between a first rotatable support (62) insertable in the lid (20) and a second rotatable support (63) insertable in the base (14) of the culture tank (10).

4. The apparatus (1) according to one of the preceding claims, wherein the second lighting means (60) comprise eight second light sources (61) positioned equidistant from each other and extending along the main body (12) of the culture tank (10).

5. The apparatus (1) according to one of the preceding claims, wherein the first lighting means (20) comprise a plurality of first light sources (53), preferably three, attachable to a longitudinal structure, preferably with a triangular cross-section, insertable inside the culture tank (10).

6. The apparatus (1) according to one of the preceding claims, wherein the main body (12) has a cylindrical shape with a diameter that ranges between 550 mm and 450 mm and the first lighting means (20) are inserted in a container (51) extending along the longitudinal axis (13) of the main body (12), said container (51) having a cylindrical shape with a diameter that ranges between 250 mm and 150 mm.

7. The apparatus (1) according to claim 6, wherein the container (51) inside of which the first lighting means (20) are inserted comprises an upper anchorage point, at the second end (122) of the main body (12), and a lower anchorage point, at the drainage opening (141).

8. The apparatus (1) according to one of the preceding claims wherein the first lighting means (20) are arranged parallel to the second lighting means (60) along the longitudinal axis (13) and wherein the distance between the first lighting means (20) and the second lighting means (60) ranges between 200 mm and 100 mm, and is preferably 150 mm.

9. The apparatus (1) according to one of the preceding claims, also comprising a plurality of reflective panels (64) joined to the second lighting means (60) extending along the main body (12) of the culture tank (10) and fixed between a seat positioned in the lid (20) and a seat (147) positioned in the base (14) of the culture tank (10).

10. The apparatus (1) according to any one of the preceding claims, also comprising a plurality of sliding reflective panels (65) extending along the main body (12) of the culture tank (10) and sliding along the outer perimeter of said main body (12) and wherein the second lighting means (60) are positioned in a space between said sliding reflective panels (65) and said outer perimeter of the main body (12).

11. The apparatus (1) according to one of the preceding claims, wherein the base (14) of the culture tank (10) has a hemispheric shape with the drainage opening (141) centrally positioned on the apex of the hemisphere (143) and comprising a plurality of nozzles (142) for coupling with the compression unit (30) provided with an autonomous resistor (35), said nozzles (142) being arranged radially with respect to said drainage opening (141) and tilted by an angle α in relation to the longitudinal axis (13) of the main body (12) of the culture tank (10).

12. The apparatus (1) according to claim 10, also comprising an air separator (70) positioned between the compression unit (30) and the base (14) of the culture tank (10) connected to the control unit (40) to allow the sequential introduction of gas inside the culture tank (10) through a selected plurality of nozzles (142).

13. The apparatus (1) according to one of the preceding claims, wherein the compression unit (30) comprises a first inlet (301) for introducing atmospheric air, a second inlet (302) for introducing carbon dioxide and at least one valve with electronic control connected to the control unit (40) for adjusting the amount of atmospheric air and/or carbon dioxide introduced into the culture tank (10).

14. A modular plant (100) for growing photosynthetic microorganisms comprising one or more autonomous apparatuses (1) according to one of the preceding claims wherein each apparatus (1) is provided with an inlet water connection (110) for introducing water inside the culture tank (10) directly through the inlet opening (22) of the lid (20), an outlet water connection (120) for draining the culture tank (10) through the drainage opening (141) of the base (14) and an electrical connection (130) for supplying power to the control unit (40).

15. A use of one or more autonomous apparatuses (1) according to one of the claims between 1 and 13 for cultivating photosynthetic microorganisms such as spirulina, chlorella, haematococcus pluvialis, or the like.
